# EUROPÄISCHE PATENTSCHRIFT

(11) **EP 1 052 248 B1**
(45) Veröffentlichungstag und Bekanntmachung des Hinweises auf die Patenterteilung: **19.03.2003**
(21) Anmeldenummer: 00108500.0
(22) Anmeldetag: 19.04.2000
(51) Int. Cl.: C07C 245/20, C07C 311/32

(54) **Verfahren zur Herstellung von wässrigen Diazoniumsalzlösungen**
Process for the preparation of aqueous solutions of diazonium salts
Procédé pour préparer des solutions aqueuses de sels de diazonium

(30) Priorität: 08.05.1999 DE 19921498
(43) Veröffentlichungstag der Anmeldung: 15.11.2000
(73) Patentinhaber: Clariant GmbH, 65929 Frankfurt am Main (DE)
(72) Erfinder: Nestler, Bernd, Dr., 65929 Frankfurt am Main (DE)

(56) Entgegenhaltungen:
- DE-A- 4 316 923
- FR-A- 1 431 815
- US-A- 5 051 131

## Beschreibung

Diese Erfindung betrifft ein neues Verfahren zur Herstellung von wäßrigen Diazoniumsalzlösungen. Wäßrige Diazoniumsalzlösungen werden z. B. für die Herstellung von Azoverbindungen, insbesondere von Farbstoffen und Pigmenten, verwendet. In der Literatur sind nur wenige Methoden für die Herstellung von wäßrigen Diazoniumsalzlösungen, ausgehend von aromatischen Nitroverbindungen beschrieben, auch wurden diese lediglich zur Synthese kleiner Mengen für wissenschaftliche Zwecke erarbeitet.

Üblicherweise wird die aromatische Nitroverbindung zunächst zu dem entsprechenden aromatischen Amin reduziert und nach Aufarbeitung und Isolierung, in einem zweiten, getrennten Schritt mit einem Erdalkalinitrit zu dem gewünschten Diazoniumsalz umgesetzt. Dabei erfolgt die Reduktion üblicherweise als katalytische Hydrierung unter Verwendung eines geeigneten Katalysators. Als Lösungsmittel finden in aller Regel organische Lösungsmittel, insbesondere niedermolekulare Alkohole wie Methanol, Ethanol oder Propanol, sowie entsprechende Ester, wie z.B. Essigsäureethylester, Verwendung. Nach Isolierung des dabei gebildeten aromatischen Amins wird dieses in wäßriger Lösung mit Nitrit zum entsprechenden Diazoniumsalz umgesetzt.

Ein derartiges Vorgehen ist in US-A-5 051 131 aufgezeigt. Hierin werden durch Umsetzung von Nitrobenzoylchlorid- und Nitrobenzolsulfonylchlorid-Derivaten mit aliphatischen Bisaminen hergestellte aromatische Nitroverbindungen in ethanolischer Lösung mit einem Palladium-Katalysator zum entsprechenden aromatischen Amin reduziert und letzteres aus der Lösung isoliert. Im Folgeschritt wird das isolierte Amin in essigsaurer, wäßriger Lösung mit Natriumnitrit zu der gewünschten Diazoniumsalzlösung umgesetzt. Nachteilig bei dieser Vorgehensweise ist die Verwendung von verschiedenen Lösungsmitteln in den beiden Reaktionsstufen, da nach der ersten Stufe das eingesetzte organische Lösungsmittel in einem separaten Schritt abgetrennt werden muß und als Abfallprodukt anfällt. Aufgrund der Brennbarkeit und möglichen Bildung von zündfähigen Lösungsmitteldampf/Luft-Gemischen ist dieser Schritt technisch aufwendig und zudem auch aus sicherheitstechnischer Sicht bedenklich.

Dieser Erfindung liegt die Aufgabe zugrunde, ein einfaches Verfahren zur Herstellung wäßriger Lösungen von Diazoniumsalzen der allgemeinen Formel (1), ausgehend von aromatischen Nitroverbindungen der allgemeinen Formel(2), zu finden. Desweiteren soll das erfindungsgemäße Verfahren sicherheitstechnisch bedenkliche Lösungsmittel vermeiden und die entstehenden Produkte sollen in hoher Ausbeute mit minimalen Abfallmengen anfallen.

Überraschenderweise wurde gefunden, daß, wenn die aromatische Nitroverbindung der allgemeinen Formel (2) in ein wasserlösliches Salz überführt wird oder als ein solches vorliegt, dieses in wäßriger Lösung zu einem Salz des wäßrigen aromatischen Amins der allgemeinen Formel (3) reduziert werden kann, die, gegebenenfalls nach Entfernen des Katalysators, direkt mit einem Diazotierungsreagenz, wie z. B. einem Nitrit, in die gewünschte wäßrige Diazoniumsalzlösung überführt werden kann, ohne daß ein Lösemittelwechsel notwendig ist

Gegenstand der Erfindung ist ein Verfahren zur Herstellung wäßriger Lösungen von Diazoniumsalzen der allgemeinen Formel (1) 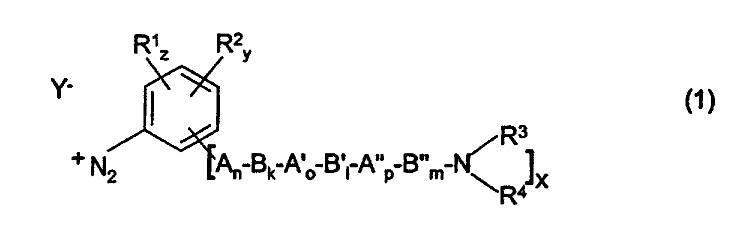 worin die Aminogruppen teilweise oder vollständig in protonierter Form vorliegen, R¹, R², R³ und R⁴ unabhängig voneinander Wasserstoff, einen C₁- bis C₁₆-Alkyl-, C₃-C₇-Cycloalkyl-, C₂-C₁₆-Alkenyl-, Aryl- oder einen 5- bis 6-gliedrigen aliphatischen oder aromatischen Heterocyclus bedeuten, die gegebenenfalls durch eine oder mehrere, z.B. 1,2 oder 3, C₁-C₄-Alkylreste, Halogenatome oder Gruppen der Formel =O, -OR', -NR'R", -SR', -COR', -COOR',-CONR'R", -NR'CONR''R''' oder -SO₂NR'R'' substituiert sind, wobei R', R", R"' für Wasserstoff oder einen C₁- bis C₁₀-Alkyl-, C₃-C₇-Cycloalkyl-, C₂-C₁₆-Alkenyl-, Aryl- oder einen 5- bis 6-gliedrigen aliphatischen oder aromatischen Heterocyclus stehen, oder wobei R' und R", oder R" und R''', oder R³ und R⁴ zusammen mit dem benachbarten N-Atom einen 5- oder 6-gliedrigen aliphatischen oder aromatischenHeterocyclus bilden, und
R¹ und R² zusätzlich für Halogen oder Gruppen der Formel -OR', -NR'R'', -SR', -COR', -COOR', -CONR'R", -SO₃H, -PO₄H₂, -NR'CONR"R''' oder -SO₂NR'R" stehen können;
oder R¹, R², R³ und R⁴ unabhängig voneinander für ein mit 1 bis 3 Sulfogruppen substituiertes C₁-C₆-Alkyl, C₅-C₆-Cycloalkyl, C₂-C₆-Alkenyl, C₆-C₁₀-Aryl oder Pyridinyl stehen;
A, A' und A" unabhängig voneinander eine divalente Gruppe der Formel -O-, -NR'-, -S-, -CO-, -COO-, -CONR'-, -NR'CONR"-oder-SO₂NR'bedeuten;
B, B' und B'' unabhängig voneinander für einen C₁- bis C₁₄-Alkylen-, C₃-C₇-Cycloalkylen-, C₂-C₁₄-Alkenylen-, Arylen- oder Heteroarylenrest steht,
oder mit 1 bis 4 Resten aus der Gruppe C₁-C₄-Alkyl, C₁-C₄-Alkoxy, OH, NH₂, NH(C₁-C₄alkyl), N(C₁-C₄-alkyl)₂, COOH, CONH₂, CONH(C₁-C₄-alkyl), CON(C₁-C₄-alkyl)₂, F und Cl substituiertes C₂-C₆-Alkylen, C₆-C₁₀-Arylen, Benzylen oder Pyridylen bedeuten;
oder B, B' oder B" bilden zusammen mit R³ oder R⁴ einen Heterocyclus aus der Gruppe Pyrrolidin, Piperidin, Piperazin und Morpholin, der zgl. mit 1 bis 6 Resten aus der Gruppe C₁-C₄-Alkyl, C₁-C₄-Alkoxy, OH, NH₂, NH(C₁-C₄-alkyl), N(C₁-C₄-alkyl)₂, COOH, CONH₂, CONH(C₁-C₄-alkyl), CON(C₁-C₄-alkyl)₂, F und Cl substituiert ist,
Y⁻ für F⁻, Cl⁻, Br, HSO₄⁻, ½ SO₄²⁻, H₂PO₄⁻, ½ HPO₄²⁻, ¹/₃ PO₄³⁻, ein Anion einer pyro-, meta- oder poly-Phosphorsäure, BF₄⁻, PF₆⁻, HCO₃⁻, ½ CO₃²⁻, NO₂⁻, NO₃⁻, Formiat, Acetat, Propionat, R''''SO₄⁻, R''''SO₃⁻ oder R''''COO⁻ steht, wobei R'''' einen C₁-C₂₁-Alkyl- oder Arylrest, vorzugsweise C₁-C₁₀-Alkyl, Naphthyl oder Phenyl bedeutet;
k, l und m unabhängig voneinander die Zahl 0, 1, 2 oder 3 sind;
n, o und p unabhängig voneinander für die Zahlen 0 oder 1 stehen;
x eine ganze Zahl von 1 bis 3; und
y und z jeweils eine ganze Zahl von 1 bis 3 bedeuten, wobei die Summe aus x, y und z 3 bis 5 ist,
dadurch gekennzeichnet, daß man
eine wäßrige Lösung eines wasserlöslichen Satzes der aromatischen Nitroverbindung der allgemeinen Formel (2), 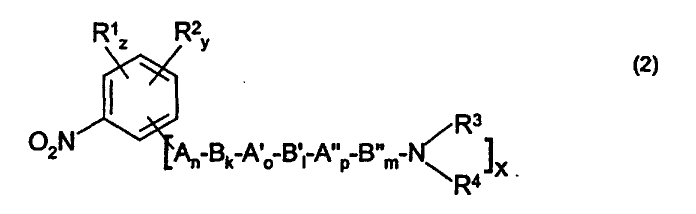 mit Wasserstoff und in Gegenwart eines Katalysators, der die Reduktion von aromatischen Nitrogruppen zu Aminogruppen katalysiert, umsetzt und das dabei entstandene aromatische Amin in der wäßrigen Lösung mit einem Diazotierungsreagenz zum Diazoniumsalz diazotiert.

In den vorstehenden Definitionen steht Aryl bevorzugt für Phenyl oder Naphthyl. Bevorzugt sind Verbindungen der Formel (1) und (2), worin A, A' und A" für -O-, -NR'-, -CONR' oder -SO₂NR'- stehen.
Weiterhin bevorzugt sind Verbindungen der Formel (1) und (2), worin B, B' und B" für unsubstituiertes oder mit 1 bis 4 Resten aus der Gruppe C₁-C₄-Alkyl, C₁-C₄-Alkoxy, OH, NH₂, NH(C₁-C₄-alkyl), NH(C₁-C₄-alkyl)₂, COOH, CONH₂, CONH(C₁-C₄alkyl), CON(C₁-C₄-alkyl)₂, F und Cl substituiertes C₂-C₆-Alkylen, C₆-C₁₀-Arylen, Benzylen oder Pyridylen stehen, oder zusammen mit R³ und/oder R⁴ einen Heterocyclus aus der Gruppe Pyrrolidin, Piperidin, Piperazin und Morpholin bilden, der gegebenenfalls mit 1 bis 6 Resten aus der Gruppe C₁-C₄-Alkyl, C₁-C₄-Alkoxy, OH, NH₂, NH(C₁-C₄-alkyl), NH(C₁-C₄-alkyl)₂, COOH, CONH₂, CONH(C₁-C₄-alkyl), CON(C₁-C₄-alkyl)₂, F und Cl substituiert ist.

Weiterhin bevorzugt sind Verbindungen der Formel (1) und (2), worin R¹ und R² C₁-C₆-Alkyl, C₅-C₆-Cycloalkyl, C₂-C₆-Alkenyl, C₆-C₁₀-Aryl oder Pyridinyl bedeuten, die gegebenenfalls durch 1 bis 3 Reste aus der Gruppe Fluor, Chlor, Brom, C₁-C₄-Alkoxy, NH₂, N(C₁-C₄-alkyl)₂,NH(C₁-C₄-alkyl), COH, CO(C₁-C₄-alkyl), COOH, -COO(C₁-C₄-alkyl), CONH₂, CONH(C₁-C₄-alkyl), CON(C₁-C₄-alkyl)₂, SO₂NH₂, SO₂NH(C₁-C₄-alkyl), SO₂N(C₁-C₄-alkyl)₂ und SO₃H substituiert sind; oder Wasserstoff, Fluor, Chlor, Brom, C₁-C₄-Alkoxy, NH₂, NH(C₁-C₄-alkyl), N(C₁-C₄-alkyl)₂, S(C₁-C₄-alkyl), CO(C₁-C₄-alkyl), COOH, COO(C₁-C₄-alkyl), CONH₂, CONH(C₁-C₄alkyl), CON(C₁-C₄-alkyl)₂, SO₃H, SO₂NH₂, SO₂NH(C₁-C₄-alkyl) oder SO₂N(C₁-C₄alkyl)₂ bedeuten.

Weiterhin bevorzugt sind Verbindungen der Formel (1) und (2), worin R³ und R⁴ C₁-C₆-Alkyl, C₅-C₆-Cycloalkyl, C₂-C₆-Alkenyl, C₆-C₁₀-Aryl oder Pyridinyl bedeuten, die gegebenenfalls durch 1 bis 3 Reste aus der Gruppe Fluor, Chlor, Brom, C₁-C₄-Alkoxy, NH₂, N(C₁-C₄-alkyl)₂,NH(C₁-C₄-alkyl), COH, CO(C₁-C₄-alkyl), COOH, -COO(C₁-C₄-alkyl), CONH₂, CONH(C₁-C₄-alkyl), CON(C₁-C₄-alkyl)₂, SO₂NH₂, SO₂NH(C₁-C₄-alkyl), SO₂N(C₁-C₄-alkyl)₂ und SO₃H substituiert sind; oder Wasserstoff bedeuten.

Weiterhin bevorzugt sind Verbindungen der Formel (1) und (2), worin m und p Null bedeuten.

Weiterhin bevorzugt sind Verbindungen der Formel (1) und (2), worin x die Zahl 1 oder 2 bedeutet.

Weiterhin bevorzugt sind Verbindungen der Formel (1), worin Y⁻ Cl⁻, HSO₄⁻, ½ SO₄²⁻, H₂PO₄⁻, ½ HPO₄²⁻, ¹/₃ PO₄³⁻, ein Anion der Pyro-, Meta- oder Poly-Phosphorsäure, Acetat oder Propionat bedeutet.

Die erfindungsgemäß verwendeten aromatischen Nitroverbindungen der Formel (2) umfassen auch davon abgeleitete Salze, in denen die Aminogruppe(n) in protonierter Form vorliegt und ein oder mehrere Gegenionen, wie z.B. F⁻, Cl⁻, Br⁻, HSO₄⁻, SO₄²⁻, H₂PO₄⁻, HPO₄²⁻, PO₄³⁻, Anionen der Pyro-, Meta- oder Poly-Phosphorsäure, BF₄⁻, PF₆⁻, HCO₃⁻, CO₃²⁻, NO₂⁻, NO₃⁻, Formiat-, Acetat- oder Propionat sowie Anionen des Typs R''''SO₄⁻, R''''SO₃⁻, R''''COO⁻, wobei R"" wie vorstehend definiert ist, vorhanden sind. Bevorzugt sind hierbei F⁻, Cl⁻, HSO₄⁻, SO₄²⁻, H₂PO₄⁻, HPO₄²⁻, PO₄³⁻, NO₃⁻, Acetat oder Propionat-lonen.

Als aromatische Nitroverbindungen werden vorzugsweise solche Verbindungen eingesetzt, die in ein wasserlösliches Salz überführt werden können und als solches hydrolysestabil sind. Beispiele für derartige Verbindungen sind:
ortho-, meta- und para-N,N-Dimethylnitroanilin,
ortho-, meta- und para-N,N-Diethylnitroanilin,
ortho-, meta- und para-N,N-Dipropylnitroanilin,
ortho-, meta- und para-Nitrobenzoesäure-2-dimethylaminoethylamid,
ortho-, meta- und para-Nitrobenzoesäure-2-diethylaminoethylamid,
ortho-, meta- und para-Nitrobenzoesäure-2-dipropylaminoethylamid,
ortho-, meta- und para-Nitrobenzoesäure-2-dibutylaminoethylamid,
ortho-, meta- und para-Nitrobenzoesäure-3-dimethylaminopropylamid,
ortho-, meta- und para-Nitrobenzoesäure-3-diethylaminopropylamid,
ortho-, meta- und para-Nitrobenzoesäure-3-dipropylaminopropylamid,
ortho-, meta- und para-Nitrobenzoesäure-3-dibutylaminopropylamid,
ortho-, meta- und para-N-(Piperidin-4-yl)nitrobenzamid,
ortho-, meta- und para-N-(2,2,6,6-Tetramethyl-piperidin-4-yl)nitrobenzamid,
N-(2,2,6,6-Tetramethyl-piperidin-4-yl)-3-nitrobenzamid,
N-(2,2,6,6-Tetramethyl-piperidin-4-yl)-4-methyl-3-nitrobenzamid,
4-Methyl-1-(4-nitrobenzoyl)piperazin,
N-(2-Morpholin4-yl-ethyl)-4-nitrobenzamid,
N-(2-Imidazol-1-yl-ethyl)-4-Nitrobenzamid,
1-(2-Nitrobenzoyl)-4,7,7-trimethyl-diethylentriamin,
1-(3-Nitrobenzoyl)-4,7,7-trimethyl- diethylentriamin,
1-(4-Nitrobenzoyl)-4,7,7-trimethyl- diethylentriamin,
1-(4-Nitrobenzoyl)-4,7,7-triethyl- diethylentriamin,
ortho-, meta- und para-Nitrobenzolsulfonsäure-2-dimethylaminoethylamid,
ortho-, meta- und para-Nitrobenzolsulfonsäure-2-diethylaminoethylamid,
ortho-, meta- und para-Nitrobenzolsulfonsäure-2-dipropylaminoethylamid,
ortho-, meta- und para-Nitrobenzolsulfonsäure-2-dibutylaminoethylamid,
ortho-, meta- und para-Nitrobenzolsulfonsäure-3-dimethylaminopropylamid,
ortho-, meta- und para-Nitrobenzolsulfonsäure-3-diethylaminopropylamid,
ortho-, meta- und para-Nitrobenzolsulfonsäure-3-dipropylaminopropylamid,
ortho-, meta- und para-Nitrobenzolsulfonsäure-3-dibutylaminopropylamid,
N-2-Dimethylamino-ethyl-N'-(4-nitrophenyl)-harnstoff,
N-2-Diethylamino-ethyl-N'-(4-nitrophenyl)-harnstoff,
N-2-Diethylamino-ethyl-N'-(3-nitrophenyl)-hamstoff,
N-3-Dimethylamino-propyl-N'-(4-nitrophenyl)-hamstoff,
N-3-Diethylamino-propyl-N'-(4-nitrophenyl)-harnstoff,
Dimethyl-2-(2-nitrophenoxy)ethylamin,
Diethyl-2-(2-nitrophenoxy)ethylamin,
Dipropyl-2-(2-nitrophenoxy)ethylamin,
Dimethyl-3-(3-nitrophenoxy)propylamin,
Diethyl-3-(3-nitrophenoxy)propylamin,
Dipropyl-3-(3-nitrophenoxy)propylamin,
1-Methyl-4-(4-nitrophenoxy)-piperidin,
1-Ethyl-4-(3-nitrophenoxy)-piperidin,
3-(4-nitrophenoxy)-propylmorpholin,
4-Dimethylamlno-1-(4-nitrophenyl)-butan-1-on,
4-Nitrophenyl-4-oxo-butylpiperidin,
ortho-, meta- und para-Nitrobenzoesäure-2-dimethylaminoethylester,
ortho-, meta- und para-Nitrobenzoesäure-2-diethylaminoethylester,
ortho-, meta- und para-Nitrobenzoesäure-3-dimethylaminopropylester,
ortho-, meta- und para-Nitrobenzoesäure-3-diethylaminopropylester,
N,N,N'-Trimethyl-N'-nitrophenyl-phenylendiamin,
N,N-Diethyl-N'-methyl-N'-nitrophenyl-phenylendiamin,
Nitro-N,N,N',N'-Tetramethyl-carbamoylmethylisophthalamid.

Für das erfindungsgemäße Verfahren ist es unerheblich, auf welchem Weg die wäßrige Lösung des wasserlöslichen Salzes der aromatischen Nitroverbindung der allgemeinen Formel (2) hergestellt wird. Zweckmäßigerweise wird diese durch Lösen des entsprechenden Salzes in Wasser, oder durch Umsetzung der aromatischen Nitroverbindung mit Säure zu dem entsprechenden Salz hergestellt. Dabei ist es unwesentlich, ob die Nitroverbindung zur Säure oder die Säure zur Nitroverbindung zugegeben wird. Zweckmäßigerweise wird diese Umsetzung in wäßriger Lösung durchgeführt, doch ist es auch möglich, diese in organischen oder mehrphasigen Lösemittelsystemen, d.h. Mischungen aus Wasser und organischen Lösungsmitteln, durchzuführen und das gewünschte wasserlösliche Salz der aromatischen Nitroverbindung der allgemeinen Formel (2), z. B. durch Extraktion, abzutrennen.

Die Konzentration des Salzes der aromatischen Nitroverbindung der Formel (2) in der wäßrigen Lösung kann 0,001 bis 25, vorzugsweise 0,05 bis 15, besonders bevorzugt 0,1 bis 5, Mol pro Liter Lösung sein.

Die Umsetzung der wäßrigen Lösung des wasserlöslichen Salzes der aromatischen Nitroverbindung der allgemeinen Formel (2) erfolgt durch heterogene Hydrierung mit H₂ und in Gegenwart eines geeigneten Katalysators. Geeignet sind solche Katalysatoren, die die Reduktion einer Nitrogruppe mit Wasserstoff zu einer Aminogruppe katalysieren. Beispiele für derartige Reduktionen und geeignete Katalysatoren sind z. B. in R. Schröter "Amine" Methoden der Organischen Chemie, Houben-Weyl, E. Müller (Hrsg.), Bd. XI/1 (1957), S. 360-488 gegeben. Bevorzugte Katalysatoren sind von Übergangsmetallen, insbesondere von Edelmetallen abgeleitete Hydrierkatalysatoren, wie z.B. Raney-Nickel, Cobalt, Iridium, Palladium, Platin, Rhenium, Rhodium, Ruthenium, Osmium, Zink, Legierungen dieser Metalle, Oxide dieser Metalle, wie z.B. PdO, PtO₂, Rh₂O₃, RuO₂, ZnO; weiterhin Katalysatoren, in denen diese Metalle, Legierungen oder Oxide auf geeignete Trägermaterialien, wie Kohle, Aktivkohle, Aluminiumoxid, aktiviertes Aluminiumoxid, Silicate, Ca-, Sr- und Ba-carbonat und -sulfat aufgebracht sind.

Die Konzentration des Katalysators liegt üblicherweise im Bereich von 0,0001 bis 25 Gew.-%, bevorzugt 0,001 bis 20 Gew.-%, besonders bevorzugt 0,005 bis 15 Gew. %, bezogen auf das Gewicht der Verbindung der Formel (2).

Die Konzentration der Verbindung der Formel (2) liegt bei der Umsetzung mit Wasserstoff zweckmäßigerweise im Bereich von 0,001 bis 25 mol pro Liter Lösung, bevorzugt 0,05 bis 15 mol/l, besonders bevorzugt 0,1 bis 5 mol/l. Die Umsetzung mit Wasserstoff kann unter Normaldruck durchgeführt werden. Um die Reaktion zu beschleunigen, kann diese auch unter erhöhtem H₂-Druck durchgeführt werden, z.B. von 1 bis 200 bar, vorzugsweise von 2 bis 150 bar, insbesondere von 5 bis 100 bar. Die Reaktion erfolgt zweckmäßigerweise bei Temperaturen zwischen 0 und 250°C, bevorzugt bei 10 bis 140°C. Der hierfür benötigte Zeitraum beträgt, in Abhängigkeit von Druck, Temperatur, Katalysator und Konzentrationsverhältnissen, etwa 5 Minuten bis 3 Tage.

Nach erfolgter Umsetzung mit Wasserstoff wird zweckmäßigerweise der Katalysator abgetrennt, z. B. durch Filtration, um erneut verwendet werden zu können, doch ist es auch möglich, diesen in der Reaktionslösung zu belassen.

Die aus der Umsetzung mit Wasserstoff resultierende wäßrige Lösung wird, ggf. nach Aufkonzentrieren oder Verdünnen, mit einem Diazotierungsreagenz versetzt. Eine Übersicht über geeignete Diazotierungsreagentien und entsprechende Verfahren zu deren Umsetzung ist z.B. in R. Pütter "Stickstoff-Verbindungen I" Methoden der Organischen Chemie, Houben-Weyl, R. Stroh (Hrsg.), Bd. X/3 (1965), S. 1-66 und A. Engel "Organo-Stickstoff-Verbindungen I" Methoden der Organischen Chemie, Houben-Weyl, D. Klamann (Hrsg.), Bd. E 16a (1990), S. 1060-1079, und der dort zitierten Literatur gegeben.

Zwar ist es für das erfindungsgemäße Verfahren unerheblich, welche Diazotierungsreagentien verwendet werden, doch sind solche Reagentien vorteilhaft, die in wäßriger Lösung eingesetzt werden können. Beispiele für derartige Diazotierungsreagentien sind Alkali- und Erdalkalinitrite, Nitrosylschwefelsäure, nitrose Gase oder organische Nitrite. Bevorzugt sind hierbei Alkalinitrite, Nitrosylschwefelsäure und nitrose Gase, besonders bevorzugt sind Natriumnitrit und Nitrosylschwefelsäure, sowie wäßrige Lösungen davon.

Die Konzentration der zu diazotierenden Aminoverbindung liegt zweckmäßigerweise im Bereich von 0,001 bis 25 mol pro Liter Lösung, bevorzugt 0,05 bis 15 mol/l, besonders bevorzugt 0,1 bis 5 mol/l.

Die Diazotierungsreagentien können in stöchiometrischer Menge eingesetzt werden, doch kann es auch vorteilhaft sein, diese im Unter- oder Überschuß einzusetzen. Bevorzugt ist der Einsatz von 0,8 bis 2 Äquivalenten, besonders bevorzugt von 0,95 bis 1,3 Äquivalenten, an Diazotierungsreagenz.
Aufgrund der thermischen Labilität von Diazoniumsalzen wird die Umsetzung mit dem Diazotierungsreagenz im allgemeinen im Temperaturbereich von -15°C bis +30°C, vorzugsweise im Bereich von -5°C bis +15°C durchgeführt. Zweckmäßig ist hierbei insbesondere eine Kühlung durch Zusatz von Eis, doch kann alternativ oder zusätzlich auch eine indirekte Kühlung, d.h. durch Einsatz von Wärmetauschern erfolgen.

Da unter anderem die Geschwindigkeit der Umsetzung mit Diazotierungsreagentien sowie die Stabilität von Diazoniumsalzen vom pH-Wert abhängig sind, kann es sich als vorteilhaft erweisen, diesen durch Säure-, Basen- oder Puffer-Zusatz vor, während und/oder nach der Umsetzung mit dem Diazotierungsreagenz auf geeignete Werte einzustellen.
Geeignet sind hierbei insbesondere Mineralsäuren, niedermolekulare organische Säuren, Alkalihydroxide bzw. -laugen, Phosphat- und Acetatpuffer; bevorzugt sind Salzsäure, Schwefelsäure, Phosphorsäure, Essigsäure, Natronlauge, Kalilauge, Mischungen aus diesen Säuren oder Laugen, sowie davon abgeleitete Puffergemische.
Bevorzugt sind hierbei pH-Werte von 0 bis 9, insbesondere pH-Werte von 0,5 bis 6.

Nach Beendigung der Diazotierungsreaktion kann die erhaltene wäßrige Diazoniumsalzlösung der allgemeinen Formel (1) direkt weiterverwendet werden. Doch kann auch ein evtl. vorhandener Überschuß an Diazotierungsreagenz in der Reaktionslösung durch geeignete Reagentien, wie z. B. Amidosulfonsäure, entfernt werden. Auch kann es zweckmäßig sein, evtl. vorhandene unlösliche Verunreinigungen durch Filtration abzutrennen.

Das erfindungsgemäße Verfahren zeichnet sich durch die Vermeidung organischer Lösungsmittel aus, so daß kein Schritt zu deren Abtrennung notwendig ist und diese nicht als Abfall anfallen. Zudem werden sehr gute Ausbeuten erzielt.

In den nachfolgenden Beispielen bedeuten Prozentangaben Gewichtsprozente.

### Beispiel 1: Synthese von wäßriger 4-(3-Diethylaminopropyl)carbamoyl-benzoldiazoniumchlorid-Lösung in methanolischer Lösung (Vergleichsbeispiel).

315 g 4-Nitrobenzoesäure-3-diethylaminopropylamid-Hydrochlorid (998 mmol) wurden in 2,8 1 Methanol gelöst, 28 g Palladium-Katalysator (5 % Palladium auf Aktivkohle, 55 % Wassergehalt) zugesetzt und 3 h bei einem Druck von 50 bar unter einer Wasserstoffatmosphäre gerührt. Der Katalysator wurde abfiltriert und von der erhaltenen Lösung das Solvens abdestilliert. Es wurden 310 g eines zähflüssigen Öls erhalten. 93,3 g dieses Öls (ca. 0,3 mol) wurden in 300 ml Wasser und 93 ml Salzsäure (31 %) gelöst, mit Eis auf 0 °C gekühlt und innerhalb von 5 Minuten mit 36 ml Natriumnitritlösung (40 %) (251 mmol) versetzt. Es wurde 30 Minuten nachgerührt, anschließend wurde der Nitritüberschuß durch Zusatz von Amidosulfonsäure entfernt (negativer Nitrittest mit Diphenylsulfon). Erhalten wurden 800 ml 8,2 gew.-%ige Diazoniumsalzlösung. Ausbeute 84 %.

### Beispiel 2: Synthese von wäßriger 4-(3-Diethylaminopropyl)carbamoyl-benzoldiazoniumchlorid-Lösung in wäßriger Lösung

Zu einer Lösung von 63,2 g 4-Nitrobenzoesäure-3-diethylaminopropylamid-Hydrochlorid (200 mmol) in 500 ml Wasser wurden 6,00 g Palladium-Katalysator (5 % Palladium auf Aktivkohle, 57 % Wassergehalt) zugesetzt und unter einer Wasserstoffatmosphäre bei einem Druck von 50 bar bis zum Ende der Wasserstoffaufnahme gerührt (Dauer ca. 0,5 h). Der Katalysator wurde abfiltriert, die erhaltene Lösung mit 62 ml Salzsäure (31 %) versetzt, mit Eis auf 0°C gekühlt und innerhalb von 5 min mit 25,0 ml Natriumnitritlösung (40 %) (189 mmol) diazotiert. Nach 30 min Rühren wurde der Nitritüberschuß durch Zusatz von Amidosulfonsäure entfernt (negativer Nitrittest mit Diphenylsulfon). Erhalten wurden 850 ml 5,8 gew.-%ige Diazoniumsalzlösung. Ausbeute 95 %.

In analoger Weise wie in Beispiel 2 wurden wäßrige Lösungen folgender Diazoniumsalze hergestellt:

Beispiel 3: 3-(2-Dimethylaminoethyl)carbamoyl-benzol-diazoniumchlorid Ausbeute: 93 %

Beispiel 4: 4-[2-(2-Dimethylaminoethyl]methylaminoethyl]carbamoyl-benzoldiazoniumchlorid Ausbeute: 94 %

Beispiel 5: 4-(3-Diethylaminopropyl)sulfamoyl-benzol-diazoniumchlorid Ausbeute: 95 %

## Patentansprüche

1. Verfahren zur Herstellung wäßriger Lösungen von Diazoniumsalzen der allgemeinen Formel (1) 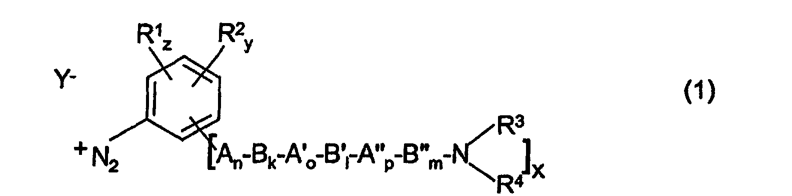 worin die Aminogruppen teilweise oder vollständig in protonierter Form vorliegen,
R¹, R², R³ und R⁴ unabhängig voneinander Wasserstoff, einen C₁- bis C₁₆-Alkyl-, C₃-C₇-Cycloalkyl-, C₂-C₁₆-Alkenyl-, Aryl- oder einen 5- bis 6-gliedrigen aliphatischen oder aromatischen Heterocyclus bedeuten, die gegebenenfalls durch eine oder mehrere C₁-C₄-Alkylreste, Halogenatome oder Gruppen der Formel =O, -OR', -NR'R", -SR', -COR', -COOR', -CONR'R", -NR'CONR"R"' oder -SO₂NR'R" substituiert sind, wobei R'. R". R"' für Wasserstoff oder einen C₁- bis C₁₀-Alkyl-, C₃-C₇-Cycloalkyl-, C₂-C₁₆-Alkenyl-, Aryl- oder einen 5- bis 6-gliedrigen aliphatischen oder aromatischen Heterocyclus stehen oder wobei R' und R", oder R" und R''', oder R³ und R⁴ zusammen mit dem benachbarten N-Atom einen 5- oder 6-gliedrigen aliphatischen oder aromatischen Heterocyclus bilden, und
R¹ und R² zusätzlich für Halogen oder Gruppen der Formel -OR', -NR'R", -SR', -COR', -COOR', -CONR'R", -SO₃H, -PO₄H₂, -NR'CONR''R''' oder -SO₂NR'R'' stehen können; oder R¹, R², R³ und R⁴ unabhängig voneinander für ein mit 1 bis 3 Sulfogruppen substituiertes C₁-C₆-Alkyl, C₅-C₆-Cycloalkyl, C₂-C₆-Alkenyl, C₆-C₁₀-Aryl oder Pyridinyl stehen;
A, A' und A" unabhängig voneinander eine divalente Gruppe der Formel -O-, -NR'-, -S-, -CO-, -COO-, -CONR'-, -NR'CONR"- oder -SO₂NR'bedeuten;
B, B' und B" unabhängig voneinander für einen C₁-C₁₄-Alkylen-, C₃-C₇-Cycloalkylen-, C₂-C₁₄-Alkenylen-, Arylen- oder Heteroarylenrest stehen; oder mit 1 bis 4 Resten aus der Gruppe C₁-C₄-Alkyl, C₁-C₄-Alkoxy, OH, NH₂, NH(C₁-C₄-alkyl), N(C₁-C₄-alkyl)₂, COOH, CONH₂, CONH(C₁-C₄-alkyl), CON(C₁-C₄-alkyl)₂, F und Cl substituiertes C₂-C₆-Alkylen, C₆-C₁₀-Arylen, Benzylen oder Pyridylen bedeuten;
oder B, B' oder B" bilden zusammen mit R³ oder R⁴ einen Heterocyclus aus der Gruppe-Pyrrolidin, Piperidin, Piperazin und Morpholin, der gegebenenfalls mit 1 bis 6 Resten aus der Gruppe C₁-C₄-Alkyl, C₁-C₄-Alkoxy, OH, NH₂, NH(C₁-C₄-alkyl), N(C₁-C₄-alkyl)₂, COOH, CONH₂, CONH(C₁-C₄-alkyl), CON(C₁-C₄-alkyl)₂, F und Cl substituiert ist;
Y⁻ für F⁻, Cl⁻, Br⁻, HSO₄⁻, ½ SO₄²⁻, H₂PO₄⁻, ½ HPO₄²⁻, ¹/₃ PO₄³⁻, ein Anion einer pyro-, meta- oder poly-Phosphorsäure, BF₄⁻, PF₆⁻, HCO₃⁻, ½ CO₃²⁻, NO₂⁻, NO₃⁻, Formiat, R''''SO₄⁻, R''''SO₃⁻ oder R''''COO⁻ steht, wobei R"" einen C₁-C₂₁-Alkyl- oder Arylrest bedeutet;
k, l und m unabhängig voneinander die Zahl 0, 1, 2 oder 3 sind;
n, o und p unabhängig voneinander für die Zahlen 0 oder 1 stehen;
x eine ganze Zahl von 1 bis 3; und
y und z jeweils eine ganze Zahl von 1 bis 3 bedeuten, wobei die Summe aus x, y und z 3 bis 5 ist,
**dadurch gekennzeichnet, dass** man
eine wäßrige Lösung eines wasserlöslichen Salzes der aromatischen Nitroverbindung der allgemeinen Formel (2), 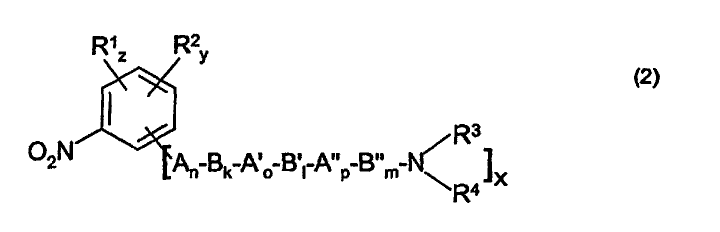 mit Wasserstoff und in Gegenwart eines Katalysators, der die Reduktion von aromatischen Nitrogruppen zu Aminogruppen katalysiert, umsetzt und das dabei entstandene aromatische Amin in der wäßrigen Lösung mit einem Diazotierungsreagenz zum Diazoniumsalz diazotiert.

2. Verfahren nach Anspruch 1, **dadurch gekennzeichnet, daß** A, A' und A" für -O-, -NR'-, -CONR'- oder -SO₂NR'- stehen.

3. Verfahren nach Anspruch 1 oder 2, **dadurch gekennzeichnet, dass** B, B' und B" für unsubstituiertes C₂-C₆-Alkylen, C₂-C₁₀-Alkylen, Benzylen oder Pyridylen stehen, oder zusammen mit R³ oder R⁴ einen Heterocyclus aus der Gruppe Pyrrolidin, Piperidin, Piperazin und Morpholin bilden.

4. Verfahren nach einem oder mehreren der Ansprüche 1 bis 3, **dadurch gekennzeichnet, dass**
R¹ und R² C₁-C₆-Alkyl, C₅-C₆-Cycloalkyl, C₂-C₆-Alkenyl, C₆-C₁₀-Aryl oder Pyridinyl bedeuten, die gegebenenfalls durch 1 bis 3 Reste aus der Gruppe Fluor, Chlor, Brom, C₁-C₄-Alkoxy, NH₂, N(C₁-C₄-alkyl)₂,NH(C₁-C₄-alkyl), COH, CO(C₁-C₄-alkyl), COOH, -COO(C₁-C₄-alkyl), CONH₂, CONH(C₁-C₄-alkyl), CON(C₁-C₄-alkyl)₂, SO₂NH₂, SO₂NH(C₁-C₄-alkyl), SO₂N(C₁-C₄-alkyl)₂ und SO₃H substituiert sind; oder Wasserstoff, Fluor, Chlor, Brom, C₁-C₄-Alkoxy, NH₂, NH(C₁-C₄-alkyl), N(C₁-C₄alkyl)₂, S(C₁-C₄-alkyl), CO(C₁-C₄-alkyl), COOH, COO(C₁-C₄-alkyl), CONH₂, CONH(C₁-C₄-alkyl), CON(C₁-C₄-alkyl)₂, SO₃H, SO₂NH₂, SO₂NH(C₁-C₄-alkyl) oder SO₂N(C₁-C₄-alkyl)₂ bedeuten.

5. Verfahren nach einem oder mehreren der Ansprüche 1 bis 4, **dadurch gekennzeichnet, dass**
R³ und R⁴ C₁-C₆-Alkyl, C₅-C₆-Cycloalkyl, C₂-C₆-Alkenyl, C₆-C₁₀-Aryl oder Pyridinyl bedeuten, die gegebenenfalls durch 1 bis 3 Reste aus der Gruppe Fluor, Chlor, Brom, C₁-C₄-Alkoxy, NH₂, N(C₁-C₄-alkyl)₂,NH(C₁-C₄-alkyl), COH, CO(C₁-C₄-alkyl), COOH, -COO(C₁-C₄-alkyl), CONH₂, CONH(C₁-C₄-alkyl), CON(C₁-C₄-alkyl)₂, SO₂NH₂, SO₂NH(C₁-C₄-alkyl), SO₂N(C₁-C₄-alkyl)₂ und SO₃H substituiert sind; oder Wasserstoff bedeuten.

6. Verfahren nach einem oder mehreren der Ansprüche 1 bis 5, **dadurch gekennzeichnet, dass** m und p Null bedeuten.

7. Verfahren nach einem oder mehreren der Ansprüche 1 bis 6, **dadurch gekennzeichnet, dass** x die Zahl 1 oder 2 bedeutet.

8. Verfahren nach einem oder mehreren der Ansprüche 1 bis 7, **dadurch gekennzeichnet, dass** die Konzentration der Verbindung der Formel (2) bei der Umsetzung mit Wasserstoff 0,001 bis 25 mol pro Liter Lösung beträgt.

9. Verfahren nach einem oder mehreren der Ansprüche 1 bis 8, **dadurch gekennzeichnet, dass** die Diazotierung bei einer Temperatur von -15°C bis +30°C durchgeführt wird.

10. Verfahren nach einem oder mehreren der Ansprüche 1 bis 9, **dadurch gekennzeichnet, dass** die Diazotierung bei einem pH-Wert zwischen 0 und 9 durchgeführt wird.

11. Verfahren nach einem oder mehreren der Ansprüche 1 bis 10, **dadurch gekennzeichnet, dass** das Diazotierungsreagenz Natriumnitrit oder Nitrosylschwefelsäure ist.

## Claims

1. A process for the preparation of aqueous solutions of diazonium salts of the formula (1) 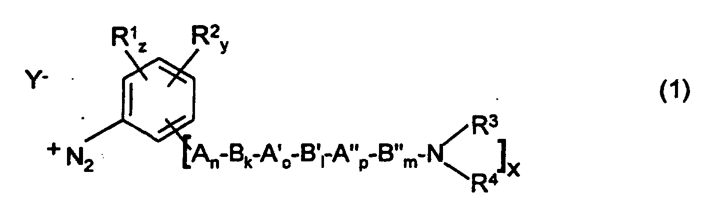 in which some or all of the amino groups are in protonated form,
R¹, R², R³ and R⁴, independently of one another, are hydrogen, a C₁-C₁₆-alkyl, C₃-C₇-cycloalkyl, C₂-C₁₆-alkenyl, aryl or a 5- to 6-membered aliphatic or aromatic heterocycle, which are optionally substituted by one or more C₁-C₄ alkyl radicals, halogen atoms or groups of the formula =O, -OR', -NR'R", -SR', -COR', -COOR', -CONR'R", -NR'CONR"R"' or -SO₂NR'R", where R', R", R"' are hydrogen or a C₁-C₁₀-alkyl, C₃-C₇-cycloalkyl, C₂-C₁₆-alkenyl, aryl or a 5- to 6-membered aliphatic or aromatic heterocycle, or where R' and R", or R" and R"', or R³ and R⁴ together with the adjacent N atom form a 5- or 6-membered aliphatic or aromatic heterocycle, and
R¹ and R² can additionally be halogen or groups of the formula -OR', -NR'R", -SR', -COR', -COOR', -CONR'R", -SO₃H, -PO₄H₂, -NR'CONR"R"' or -SO₂NR'R";
or R¹, R², R³ and R⁴, independently of one another, are a C₁-C₆-alkyl, C₅-C₆-cycloalkyl, C₂-C₆-alkenyl, C₆-C₁₀-aryl or pyridinyl substituted by 1 to 3 sulfo groups;
A, A' and A", independently of one another, are a divalent group of the formula -O-, -NR'-, -S-, -CO-, -COO-, -CONR'-, -NR'CONR"-or -SO₂NR'-;
B, B' and B", independently of one another, are a C₁-C₁₄-alkylene, C₃-C₇-cycloalkylene, C₂-C₁₄-alkenylene, arylene or heteroarylene radical, or
C₂-C₆-alkylene, C₆-C₁₀-arylene, benzylene or pyridylene substituted by 1 to 4 radicals from the group consisting of C₁-C₄-alkyl, C₁-C₄-alkoxy, OH, NH₂, NH(C₁-C₄-alkyl), N(C₁-C₄-alkyl)₂, COOH, CONH₂, CONH(C₁-C₄-alkyl), CON(C₁-C₄-alkyl)₂, F and Cl;
or B, B' or B" form, together with R³ or R⁴, a heterocycle from the group consisting of pyrrolidine, piperidine, piperazine and morpholine, which is optionally substituted by 1 to 6 radicals from the group consisting of C₁-C₄-alkyl, C₁-C₄-alkoxy, OH, NH₂, NH(C₁-C₄-alkyl), N(C₁-C₄-alkyl)₂, COOH, CONH₂, CONH(C₁-C₄-alkyl), CON(C₁-C₄-alkyl)₂, F and Cl;
Y⁻ is F⁻, Cl⁻, Br⁻, HSO₄⁻, ½SO₄²⁻, H₂PO₄⁻, ½HPO₄²⁻, ¹/₃PO₄³⁻, an anion of a pyro-, meta- or poly-phosphoric acid, BF₄⁻, PF₆⁻, HCO₃⁻, ½CO₃²⁻, NO₂⁻, NO₃⁻, formate, R''''SO₄⁻, R''''SO₃⁻ or R''''COO⁻, where R"" is a C₁-C₂₁-alkyl or aryl radical;
k, I and m, independently of one another, are the number 0, 1, 2 or 3;
n, o and p, independently of one another, are the numbers 0 or 1;
x is an integer from 1 to 3; and
y and z are each an integer from 1 to 3, where the sum x + y + z is 3 to 5, which comprises
reacting an aqueous solution of a water-soluble salt of the aromatic nitro compound of the formula (2), 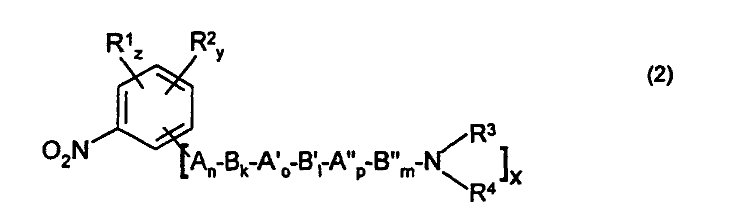 with hydrogen and in the presence of a catalyst which catalyzes the reduction of aromatic nitro groups to amino groups, and diazotizing the aromatic amine formed in the process in the aqueous solution with a diazotization reagent to give the diazonium salt.

2. The process as claimed in claim 1, wherein A, A' and A" are -O-, -NR'-, -CONR'- or -SO₂NR'-.

3. The process as claimed in claim 1 or 2, wherein B, B' and B'' are unsubstituted C₂-C₆-alkylene, C₆-C₁₀-arylene, benzylene or pyridylene, or together with R³ or R⁴ form a heterocycle from the group consisting of pyrrolidine, piperidine, piperazine and morpholine.

4. The process as claimed in one or more of claims 1 to 3, wherein R¹ and R² are C₁-C₆-alkyl, C₅-C₆-cycloalkyl, C₂-C₆-alkenyl, C₆-C₁₀-aryl or pyridinyl which are optionally substituted by 1 to 3 radicals from the group consisting of fluorine, chlorine, bromine, C₁-C₄-alkoxy, NH₂, N(C₁-C₄-alkyl)₂, NH(C₁-C₄-alkyl), COH, CO(C₁-C₄-alkyl), COOH, -COO(C₁-C₄-alkyl), CONH₂, CONH(C₁-C₄-alkyl), CON(C₁-C₄-alkyl)₂, SO₂NH₂, SO₂NH(C₁-C₄-alkyl), SO₂N(C₁-C₄-alkyl)₂ and SO₃H; or hydrogen, fluorine, chlorine, bromine, C₁-C₄-alkoxy, NH₂, NH(C₁-C₄-alkyl), N(C₁-C₄-alkyl)₂, S(C₁-C₄-alkyl), CO(C₁-C₄-alkyl), COOH, COO(C₁-C₄-alkyl), CONH₂, CONH(C₁-C₄-alkyl), CON(C₁-C₄-alkyl)₂, SO₃H, SO₂NH₂, SO₂NH(C₁-C₄-alkyl) or SO₂N(C₁-C₄-alkyl)₂.

5. The process as claimed in one or more of claims 1 to 4, wherein R³ and R⁴ are C₁-C₆-alkyl, C₅-C₆-cycloalkyl, C₂-C₆-alkenyl, C₆-C₁₀-aryl or pyridinyl which are optionally substituted by 1 to 3 radicals from the group consisting of fluorine, chlorine, bromine, C₁-C₄-alkoxy, NH₂, N(C₁-C₄-alkyl)₂, NH(C₁-C₄-alkyl), COH, CO(C₁-C₄-alkyl), COOH, -COO(C₁-C₄-alkyl), CONH₂, CONH(C₁-C₄-alkyl), CON(C₁-C₄-alkyl)₂, SO₂NH₂, SO₂NH(C₁-C₄-alkyl), SO₂N(C₁-C₄-alkyl)₂ and SO₃H; or hydrogen.

6. The process as claimed in one or more of claims 1 to 5, wherein m and p are zero.

7. The process as claimed in one or more of claims 1 to 6, wherein x is the number 1 or 2.

8. The process as claimed in one or more of claims 1 to 7, wherein the concentration of the compound of the formula (2) in the reaction with hydrogen is 0.001 to 25 mol per liter of solution.

9. The process as claimed in one or more of claims 1 to 8, wherein the diazotization is carried out at a temperature of from -15°C to +30°C.

10. The process as claimed in one or more of claims 1 to 9, wherein the diazotization is carried out at a pH between 0 and 9.

11. The process as claimed in one or more of claims 1 to 10, wherein the diazotization reagent is sodium nitrite or nitrosylsulfuric acid.

## Revendications

1. Procédé pour la préparation de solutions aqueuses de sels de diazonium de formule générale (1) 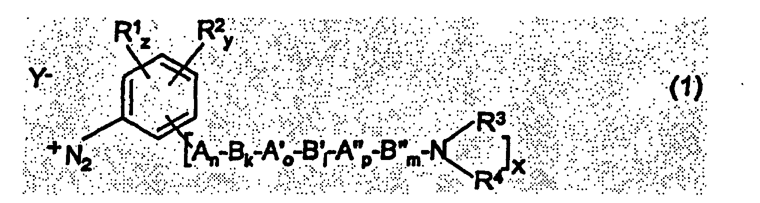 dans laquelle les groupes amino se présentent partiellement ou complètement sous forme protonée,
R¹, R², R³ et R⁴ représentent indépendamment les uns des autres un atome d'hydrogène, un groupe alkyle en C₁ à C₁₆, cycloalkyle en C₃ à C₇, alcényle en C₂ à C₁₆, aryle ou un hétérocycle à 5 à 6 chaînons aliphatique ou aromatique, qui sont éventuellement substitués par un ou plusieurs groupes alkyle en C₁ à C₄, atomes d'halogène ou groupes de formule =O, -OR', -NR'R'', -SR', -COR', -COOR', -CONR'R'', -NR'CONR''R''' ou -SO₂NR'R'', dans lesquels R', R'', R''' représentent un atome d'hydrogène ou un groupe alkyle en C₁ à C₁₀, cycloalkyle en C₃ à C₇, alcényle en C₂ à C₁₆, aryle ou un hétérocycle à 5 à 6 chaînons aliphatique ou aromatique ou dans lesquels R' et R'', ou R'' et R''', ou R³ et R⁴ forment ensemble avec l'atome d'azote voisin un hétérocycle à 5 ou 6 chaînons aliphatique ou aromatique, et
R¹ et R² représentent de plus un atome d'halogène ou des groupes de formule -OR', -NR'R'', -SR', -COR', -COOR', -CONR'R'', -SO₃H, -PO₄H₂, -NR'CONR''R''' ou -SO₂NR'R'' ; ou R¹, R², R³ et R⁴ représentent indépendamment les uns des autres un groupe alkyle en C₁ à C₆, cycloalkyle en C₅ à C₆, alcényle en C₂ à C₆, aryle en C₆ à C₁₀ ou pyridinyle substitué par 1 à 3 groupes suifo ;
A, A' et A'' représentent indépendamment les uns des autres un groupe divalent de formule -O-, -NR'-, -S-, -CO-, -COO-, -CONR¹-, -NR'CONR''- ou -SO₂NR'- ;
B, B' et B'' représentent indépendamment les uns des autres un groupe alkylène en C₁ à C₁₄, cycloalkylène en C₃ à C₇, alcénylène en C₂ à C₁₄, arylène ou hétéroarylène ; ou alkylène en C₂ à C₆, arylène en C₆ à C₁₀, benzylène ou pyridylène substitués par 1 à 4 restes du groupe alkyle en C₁ à C₄, alcoxy en C₁ à C₄, OH, NH₂, NH(alkyle en C₁ à C₄), N(alkyle en C₁ à C₄)₂, COOH, CONH₂, CONH(alkyle en C₁ à C₄), CON(alkyle en C₁ à C₄)₂, F et Cl ;
ou B, B' et B'' forment ensemble avec R³ ou R⁴ un hétérocycle choisi parmi un groupe pyrrolidine, pipéridine, pipérazine et morpholine, qui est éventuellement substitué avec 1 à 6 restes du groupe alkyle en C₁ à C₄, alcoxy en C₁ à C₄, OH, NH₂, NH(alkyle en C₁ à C₄), N(alkyle en C₁ à C₄)₂, COOH, CONH₂, CONH(alkyle en C₁ à C₄), CON(alkyle en C₁ à C₄)₂, F et Cl ;
Y⁻ représente F⁻, Cl⁻, Br⁻, HSO₄⁻, ½ SO₄²⁻, H₂PO₄⁻, ½ H₂PO₄²⁻, 1/3 PO₄³⁻, un anion d'un acide pyro-, méta- ou poly-phosphorique, BF₄⁻, PF₆⁻, HCO₃⁻, ½ CO₃²⁻, NO₂⁻, NO₃⁻, formiate, R''''SO₄⁻, R''''SO₃⁻ ou R''''COO⁻, dans lesquels R'''' représente un groupe alkyle en C₁ à C₂₁ ou aryle ;
k, l et m représentent indépendamment les uns des autres le nombre 0, 1, 2 ou 3 ;
n, o et p représentent indépendamment les uns des autres les nombres 0 ou 1 ;
x représente un nombre entier de 1 à 3 ; et
y et z représentent respectivement un nombre entier de 1 à 3, la somme de x, y et z étant de 3 à 5,
**caractérisé en ce qu'**on met à réagir une solution aqueuse d'un sel hydrosoluble du composé nitro aromatique de formule générale (2), 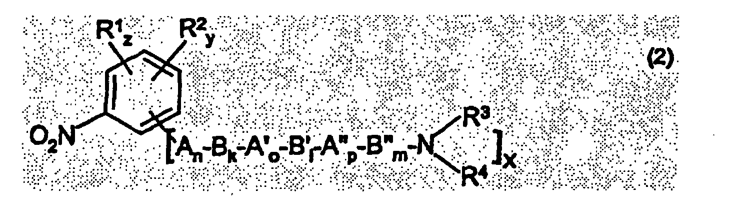 avec de l'hydrogène et en présence d'un catalyseur, qui catalyse la réduction de groupes nitro aromatiques en groupes amino et ce qu'on réalise la diazotation de l'amine aromatique ainsi produite en solution aqueuse avec un réactif de diazotation pour obtenir un sel de diazonium.

2. Procédé selon la revendication 1, **caractérisé en ce que** A, A' et A'' représentent -O-, -NR'-, -CONR'-ou -SO₂NR'-.

3. Procédé selon la revendication 1 ou 2, **caractérisé en ce que** B, B' et B'' représentent un groupe alkylène en C₂ à C₆ non substitué, arylène en C₆ à C₁₀, benzylène ou pyridylène, ou forment ensemble avec R³ ou R⁴ un hétérocycle choisi parmi un groupe pyrrolidine, pipéridine, pipérazine et morpholine.

4. Procédé selon une ou plusieurs des revendications 1 à 3, **caractérisé en ce que** R¹ et R² représentent un groupe alkyle en C₁ à C₆, cycloalkyle en C₅ à C₆, alcényle en C₂ à C₆, aryle en C₆ à C₁₀ ou pyridinyle, qui sont éventuellement substitués par 1 à 3 restes choisis parmi un atome de fluor, de chlore, de brome, un groupe alcoxy en C₁ à C₄, NH₂, N(alkyle en C₁ à C₄)₂, NH(alkyle en C₁ à C₄), COH, CO(alkyle en C₁ à C₄), COOH, -COO(alkyle en C₁ à C₄), CONH₂, CONH(alkyle en C₁ à C₄), CON(alkyle en C₁ à C₄)₂, SO₂NH₂, SO₂NH(alkyle en C₁ à C₄), SO₂N(alkyle en C₁ à C₄)₂ et SO₃H ; ou un atome d'hydrogène, de fluor, de chlore, de brome, un groupe alcoxy en C₁ à C₄, NH₂, NH(alkyle en C₁ à C₄), N(alkyle en C₁ à C₄)₂, S (alkyle en C₁ à C₄), CO(alkyle en C₁ à C₄), COOH, COO(alkyle en C₁ à C₄), CONH₂, CONH(alkyle en C₁ à C₄), CON(alkyle en C₁ à C₄)₂, SO₃H, SO₂NH₂, SO₂NH(alkyle en C₁ à C₄) ou SO₂N(alkyle en C₁ à C₄)₂.

5. Procédé selon une ou plusieurs des revendications 1 à 4, **caractérisé en ce que**
R³ et R⁴ représentent un groupe alkyle en C₁ à C₆, cycloalkyle en C₅ à C₆, alcényle en C₂ à C₆, aryle en C₆ à C₁₀ ou pyridinyle, qui sont éventuellement substitués par 1 à 3 groupes choisis parmi un atome de fluor, de chlore, de brome, un groupe alcoxy en C₁ à C₄, NH₂, N(alkyle en C₁ à C₄)₂, NH(alkyle en C₁ à C₄), COH, CO (alkyle en C₁ à C₄), COOH, -COO(alkyle en C₁ à C₄), CONH₂, CONH(alkyle en C₁ à C₄), CON(alkyle en C₁ à C₄)₂, SO₂NH₂, SO₂NH(alkyle en C₁ à C₄), SO₂N(alkyle en C₁ à C₄)₂ et SO₃H ; ou représentent un atome d'hydrogène.

6. Procédé selon une ou plusieurs des revendications 1 à 5, **caractérisé en ce que** m et p représentent zéro.

7. Procédé selon une ou plusieurs des revendications 1 à 6, **caractérisé en ce que** x représente le nombre 1 ou 2.

8. Procédé selon une ou plusieurs des revendications 1 à 7, **caractérisé en ce que** la concentration du composé de formule (2) dans la réaction avec l'hydrogène s'élève de 0,001 jusqu'à 25 moles par litre de solution.

9. Procédé selon une ou plusieurs des revendications 1 à 8, **caractérisé en ce que** la diazotation est réalisée à une température de -15 °C jusqu'à +30 °C.

10. Procédé selon une ou plusieurs des revendications 1 à 9, **caractérisé en ce que** la diazotation est réalisée à une valeur de pH entre 0 et 9.

11. Procédé selon une ou plusieurs des revendications 1 à 10, **caractérisé en ce que** le réactif de diazotation est le nitrite de sodium ou l'acide nitrosylesulfurique.
